# FASCICULE DE BREVET EUROPEEN

(11) **EP 3 551 253 B1**
(45) Date de publication et mention de la délivrance du brevet: **26.08.2020**
(21) Numéro de dépôt: 17832921.5
(22) Date de dépôt: 11.12.2017
(51) Int. Cl.: A61M 5/24, A61M 5/31, A61M 5/315, B65D 83/00, A61D 7/00

(54) **DISTRIBUTEUR MULTI-DOSES**
MEHRFACHDOSISSPENDER
MULTI-DOSE DISPENSER

(30) Priorité: 12.12.2016 FR 1662305
(43) Date de publication de la demande: 16.10.2019
(73) Titulaire: VIRBAC, 06516 Carros (FR)
(72) Inventeur: LEFEBVRE, Christelle, 06700 Saint Laurent du Var (FR); ROBIN, Michel, 06600 Antibes (FR)
(86) Numéro de dépôt international: PCT/EP2017/082229
(87) Numéro de publication internationale: WO 2018/108817

(56) Documents cités:
- WO-A1-93/11813
- WO-A1-94/07562
- US-A- 4 261 359
- US-A1- 2012 184 917
- US-B2- 8 540 124

## Description

### Domaine technique

L'invention concerne un distributeur multi-doses destiné à l'application d'un produit liquide, en particulier d'un produit vétérinaire, à la surface de la peau d'un animal.

### Art antérieur

Pour appliquer un produit liquide sur la peau d'un animal, il est connu d'utiliser des pipettes mono-doses, dites "spot-on". Une canule de la pipette est posée sur la peau de l'animal, puis le réservoir de produit est comprimé de manière à éjecter le produit hors de la pipette. Une pipette mono-dose présente l'avantage d'une hygiène parfaite, puisqu'elle n'est utilisée qu'une seule fois. Cependant, elle produit des quantités importantes d'emballages.

En outre, lorsqu'un traitement nécessite plusieurs applications successives, l'utilisation de pipettes mono-doses ne permet pas de déterminer facilement le nombre d'applications déjà effectuées. Enfin, une pipette mono-dose ne permet que la délivrance d'un même volume de produit. Il n'est en effet pas possible de fractionner la délivrance du produit contenu dans le réservoir.

US 8 540 124 divulgue un exemple de distributeur multi-doses répondant partiellement aux inconvénients posés par les pipettes mono-doses.

Il existe cependant un besoin permanent pour un distributeur multi-doses qui soit :
- ergonomique, en particulier pour l'application de compositions sur des animaux potentiellement nerveux ;
- compact, pour faciliter le stockage et le conditionnement ;
- d'un coût de fabrication limité ;
- garantissant des conditions d'hygiène parfaites ;
- limitant le risque de mauvaises manipulations, en particulier pour protéger les enfants ;
- facile à recycler ;
- propre, fiable et facile à utiliser.

Un but de la présente invention est de répondre, au moins partiellement, à ce besoin.

### Résumé de l'invention

L'invention propose un distributeur tel que défini par la revendication 1 et un procédé de fabrication d'un distributeur tel que défini par la revendication 15. Des formes préférentielles de réalisation sont définies dans les revendications dépendantes. L'invention propose un distributeur multi-doses comportant :
- un réservoir de produit, d'axe X, débouchant à une extrémité distale par un orifice de sortie et comportant une cuve ;
- un piston comportant
   - une tige de piston portant une crémaillère et,
   - à une extrémité distale de la tige de piston, une tête de piston montée coulissante dans la cuve, selon l'axe X ;
- un cliquet monté mobile, selon l'axe X, par rapport au réservoir, et configuré pour coopérer avec ladite crémaillère de manière à n'entraîner le piston, selon l'axe X, que dans un sens d'avancement du piston dans la cuve ;
- une coque contenant le cliquet et au moins une partie du piston, et, de préférence, au moins une partie du réservoir, et notamment la cuve du réservoir ;
- de préférence un capot adapté pour sélectivement obturer, dans une position fermée, un orifice d'éjection du produit hors du distributeur.

**Selon un premier aspect principal de l'invention,** le distributeur comporte un ressort disposé de manière à repousser le cliquet, selon l'axe X, dans un sens de recul opposé au sens d'avancement, le ressort formant une pièce monobloc avec le cliquet, de préférence étant venu de matière avec le cliquet.

Le nombre de pièces du distributeur en est réduit, ce qui en facilite l'assemblage et en réduit le coût de fabrication.

Notamment pour le premier aspect principal de l'invention, un distributeur selon l'invention peut encore comporter une ou plusieurs des caractéristiques optionnelles et préférées suivantes :
- le ressort est un ressort à lames ;
- le ressort comporte au moins une branche de ressort divergeant de l'axe X, suivant le sens d'avancement, c'est-à-dire s'écartant de plus en plus rapidement de l'axe X à mesure que l'on suit l'axe X;
- ladite branche de ressort s'étend dans un plan incluant l'axe X ;
- la branche de ressort s'effile en divergeant de l'axe X, suivant le sens d'avancement, de préférence en conservant une largeur constante, la largeur étant mesurée perpendiculairement au plan dans lequel s'étend ladite branche de ressort ;
- la branche de ressort, de préférence chaque branche de ressort, est disposée entre deux nervures de guidage, de préférence sensiblement parallèles, de préférence parallèles à l'axe X, configurées pour guider la déformation de la branche de ressort dans un plan parallèle à l'axe X, de préférence dans un plan passant par l'axe X ;
- les nervures de guidage sont venues de matière avec la coque ;
- la branche de ressort comporte une extrémité d'appui immobilisée par rapport au réservoir, de préférence par appui sur un épaulement de la coque ;
- le ressort comporte des première et deuxième dites branches de ressort, de préférence coplanaires, de préférence identiques, chaque branche de ressort divergeant de préférence de l'axe X, suivant le sens d'avancement, à partir d'une base commune et s'étendant, de préférence, dans un même plan incluant l'axe X ;
- la base est venue de matière avec lesdites première et deuxième dites branches de ressort, l'ensemble étant de préférence en un matériau polymère, de préférence en plastique ;
- les première et deuxième branche de ressort sont sensiblement identiques et s'étendent, de manière symétrique, par rapport à l'axe X. Avantageusement, l'action des branches de ressort ne déséquilibre pas le mouvement du piston.

**Selon un deuxième aspect principal de l'invention,** la crémaillère comporte des première et deuxième rangées de dents, lesdites rangées de dents s'étendant parallèlement à l'axe X, et le cliquet comporte des première et deuxième parties de pince disposées de manière à pincer élastiquement ladite crémaillère en prenant appui sur lesdites première et deuxième rangées de dents, respectivement.

Une telle configuration autorise un assemblage rapide. En outre, les forces exercées par les parties de pince sur les rangées de dents peuvent être équilibrées, ce qui stabilise le piston lors de son déplacement selon l'axe X.

Notamment pour le deuxième aspect principal de l'invention, un distributeur selon l'invention peut encore comporter une ou plusieurs des caractéristiques optionnelles et préférées suivantes :
- les première et deuxième rangées de dents s'étendent suivant des lignes rectilignes, de préférence sensiblement parallèles, de préférence dans un plan incluant l'axe X ;
- les première et deuxième rangées de dents sont disposées sur des faces opposées de ladite tige de piston, et sont de préférence venues de matière avec ladite tige de piston ;
- les première et deuxième rangées de dents sont de même longueur ;
- les première et deuxième rangées de dents s'étendent sur plus de 60%, plus de 80%, plus de 90%, de préférence sensiblement 100% de la longueur de la tige de piston ;
- le cliquet comporte des première et deuxième branches de cliquet, s'étendant de préférence parallèlement à l'axe X, terminées par lesdites première et deuxième parties de pince, respectivement, la tige de piston s'étendant, pour plus de 60%, plus de 80%, plus de 90%, plus de 95%, de préférence sensiblement 100% de sa longueur entre lesdites première et deuxième branches de cliquet, de préférence au moins dans la configuration initiale du distributeur ;
- la longueur des première et deuxième branches de cliquet est sensiblement égale à la longueur de la tige de piston ;
- le cliquet comporte au moins un, de préférence des premier et deuxième bras de butée, de préférence s'étendant chacun dans un plan perpendiculaire à l'axe X, de préférence coplanaires, chaque bras de butée étant de préférence fixé sur une branche de cliquet respective, et étant de préférence venue de matière avec ladite branche de cliquet respective, chaque bras de butée étant configuré pour limiter le déplacement du cliquet, selon l'axe X, dans le sens d'avancement ;
- le cliquet, le ressort et les bras de butée forment une pièce monobloc, de préférence en un matériau polymère, de préférence en plastique ;
- la branche de cliquet, de préférence chaque branche de cliquet, est disposée entre deux nervures de guidage, de préférence sensiblement parallèles, de préférence parallèles à l'axe X, configurées pour guider le déplacement de ladite branche de cliquet dans un plan parallèle à l'axe X, de préférence dans un plan passant par l'axe X ;
- lesdites nervures de guidage sont configurées pour guider le déplacement d'un ou plusieurs bras de butée dans un plan passant par l'axe X ;
- les nervures de guidage sont venues de matière avec la coque.

**Selon un troisième aspect principal de l'invention,** le distributeur comporte au moins une, de préférence deux, de préférence trois, de préférence quatre nervures de guidage du déplacement du piston, chaque nervure de guidage comportant deux grandes faces, de préférence sensiblement parallèles l'une à l'autre, reliées l'une à l'autre par un chant, chaque nervure de guidage étant configurée pour assurer un contact glissant dudit chant et/ou d'une desdites grandes faces sur le piston, de préférence sur la tige de piston.

Comme cela apparaîtra plus en détail dans la suite de la description, de telles nervures de guidage confèrent une grande compacité et limitent la consommation de matière et le poids du distributeur. En outre, elles facilitent le positionnement des différentes pièces, et notamment du piston, lors de l'assemblage du distributeur.

Notamment pour le troisième aspect principal de l'invention, un distributeur selon l'invention peut encore comporter une ou plusieurs des caractéristiques optionnelles et préférées suivantes :
- ladite au moins une nervure de guidage est conformée pour assurer un contact glissant à la fois par son chant et par une de ses grandes faces ;
- le distributeur comporte deux couples de dites nervures de guidage, les nervures de guidage étant de préférence sensiblement planes, de préférence encore toutes parallèles ;
- chaque nervure de guidage d'un dit couple de nervures de guidage est coplanaire à une nervure de guidage de l'autre couple de nervures de guidage ;
- les deux nervures de guidage d'un couple de nervures de guidage, de préférence de chaque couple de nervures de guidage, sont parallèles à l'axe X ;
- les deux nervures de guidage d'un couple de nervures de guidage, de préférence de chaque couple de nervures de guidage, présentent sensiblement la même hauteur ;
- la hauteur d'une nervure de guidage, de préférence de chaque nervure de guidage, est supérieure à 5 mm, de préférence supérieure à 10 mm et/ou inférieure 30 mm ;
- le chant d'une nervure de guidage, de préférence de chaque nervure de guidage, s'étend de manière rectiligne, parallèlement à l'axe X ;
- chaque nervure de guidage est plane et s'étend parallèlement à une deuxième nervure de guidage et dans le plan d'une troisième nervure de guidage ;
- l'écartement de deux nervures de guidage d'un couple de nervures de guidage est sensiblement égal à la largeur d'une, de préférence de chaque branche de cliquet, et/ou d'une, de préférence de chaque branche de ressort, et/ou d'une, de préférence de chaque bras de butée, la largeur étant mesurée perpendiculairement à un plan dans lequel s'étend(ent) la ou lesdites branches de cliquet et/ou la ou lesdites branches de ressort et/ou le ou lesdits bras de butée ;
- l'écartement des deux nervures de guidage d'au moins un couple de nervures de guidage, de préférence de chaque couple de nervures de guidage est sensiblement égal à la largeur d'une branche de cliquet et/ou d'une branche de ressort et/ou d'un bras de butée respectif, de manière à guider la déformation et/ou le déplacement, dans un plan parallèle à l'axe X, de ladite branche de cliquet et/ou de ladite branche de ressort et/ou dudit bras de butée, respectivement ;
- de préférence chaque branche de cliquet, et de préférence chaque branche de ressort, et de préférence chaque bras de butée est guidé entre des nervures de guidage d'un desdits couples de nervures de guidage ;
- au moins une, de préférence chaque nervure de guidage est configurée pour guider le coulissement, selon l'axe X, d'une cornière respective ménagée sur la tige de piston ;
- la face d'une dite cornière, de préférence d'une cornière quelconque, en contact glissant avec une grande face de la nervure de guidage respective, est délimitée par une arête rectiligne, de préférence parallèle à l'axe X, dont la longueur, le long de l'axe X est de préférence supérieure à 20%, à 30%, à 50%, à 60%, à 80%, à 90%, de préférence égale à sensiblement 100% de la longueur de la tige de piston ;
- au moins une, de préférence au moins deux, de préférence au moins trois, de préférence quatre dites cornières s'étendent le long d'une rangée de dents de la crémaillère ;
- au moins une, de préférence chaque nervure de guidage est venue de matière avec la coque.

**Selon un quatrième aspect principal de l'invention,** le distributeur comporte un organe de blocage désactivable, de préférence de manière irréversible, de préférence sécable, configuré pour empêcher la distribution d'un produit contenu dans le réservoir, et disposé à l'intérieur de la coque.

La disposition de l'organe de blocage à l'intérieur de la coque permet avantageusement de limiter les risques de blessure lors de sa désactivation, en particulier lorsque cette désactivation résulte d'une rupture brutale. Elle limite également les risques de dégradation involontaire de l'organe de blocage.

Notamment pour le quatrième aspect de l'invention, un distributeur selon l'invention peut encore présenter une ou plusieurs des caractéristiques optionnelles et préférées suivantes :
- l'organe de blocage est disposé de manière qu'avant sa désactivation, il immobilise le piston (blocage en position) ;
- avant désactivation, l'organe de blocage est fixé sur le piston, de préférence sur la tige de piston et, après désactivation, est libre par rapport au piston, de préférence la tige de piston, respectivement ;
- l'organe de blocage est relié au piston, de préférence à la tige de piston, par une zone d'affaiblissement, de préférence sous la forme d'une ligne d'affaiblissement, de préférence s'étendant perpendiculairement à l'axe X, la zone d'affaiblissement résultant de préférence d'une réduction locale d'au moins une dimension de la section de l'organe de blocage dans un plan parallèle à l'axe X, de préférence d'une réduction d'épaisseur ou du ménagement de trous à travers l'organe de blocage ;
- l'organe de blocage est relié à l'extrémité distale de la tige de piston, c'est-à-dire à l'extrémité de la tige de piston du côté de l'orifice de sortie ;
- avant désactivation de l'organe de blocage, la tige de piston et l'organe de blocage constituent une pièce monobloc ;
- l'organe de blocage est venu de matière avec la tige de piston, et, de préférence, est fabriqué par injection d'un matériau polymère, de préférence de plastique, dans un moule ;
- l'organe de blocage est désactivable par pression sur un organe de désactivation, disposé, au moins en partie, à l'extérieur de la coque, de préférence sous la seule pression d'un pouce ;
- l'organe de blocage est désactivable par une action adaptée pour distribuer une dose de produit, de préférence par appui, selon l'axe X, sur un bouton-poussoir ;
- le bouton-poussoir est mécaniquement couplé audit cliquet, c'est-à-dire que le déplacement du bouton-poussoir, en particulier pour distribuer une dose, entraîne celui du cliquet ;
- l'organe de blocage est désactivable sous l'effet d'une force, de préférence exercée sur le cliquet, de préférence par l'intermédiaire d'un bouton-poussoir, inférieure à 10 N, à 5 N, à 3 N, à 1 N. Dans un mode de réalisation, la désactivation de l'organe de blocage peut ainsi être à peine perceptible par l'opérateur ;
- alternativement, la force peut être supérieure à 3 N, supérieure à 5 N, voire supérieure à 10 N, ce qui est notamment utile lorsque l'organe de blocage est utilisé pour confirmer à l'opérateur que le distributeur n'a jamais été utilisé ;
- l'organe de désactivation est un bouton-poussoir, de préférence mécaniquement couplé au cliquet, de préférence mobile selon l'axe X, l'organe de blocage entravant ladite mobilité avant sa désactivation ;
- l'organe de blocage présente la forme d'une patte, de préférence la forme de deux pattes ;
- l'organe de blocage s'étend sensiblement dans un plan perpendiculaire à l'axe X ;
- l'organe de blocage comporte deux pattes, de préférence seulement deux pattes, de préférence sensiblement alignées, de préférence encore alignées selon un axe coupant l'axe X ;
- l'organe de blocage est fixé, de préférence par clipsage, sur la coque ;
- l'organe de blocage s'étend au moins partiellement entre deux nervures de guidage du piston, de préférence de la tige de piston, de préférence en contact avec lesdites nervures de guidage, qui contribuent ainsi au maintien en position de l'organe de blocage ;
- l'organe de blocage comporte un pion de butée configuré pour limiter le déplacement du cliquet selon l'axe X, dans le sens d'avancement, de préférence de manière que la course dudit cliquet soit égale à la longueur d'une ou plusieurs dents de la crémaillère, de préférence soit égale à la longueur d'une dent

**Selon un cinquième aspect principal de l'invention,** un distributeur selon l'invention comporte un clapet anti-retour, disposé de manière à obturer l'orifice de sortie et à n'autoriser qu'une sortie de produit hors du réservoir.

Le clapet anti-retour peut donc s'ouvrir lorsque le piston avance dans la cuve et se fermer dès que cet avancement cesse. Avantageusement, le clapet anti-retour isole de l'extérieur le produit contenu dans la cuve, ce qui stabilise les conditions physico-chimiques à l'intérieur de la cuve, et limite en particulier l'oxydation du produit. Le clapet anti-retour limite également toute évaporation du produit.

Le clapet anti-retour entrave, de préférence interdit une entrée d'air, et empêche donc avantageusement tout mouvement de recul du piston.

Enfin, le clapet anti-retour limite l'écoulement libre hors de l'embout d'application, ce qui permet de contrôler avec précision les doses délivrées.

Notamment pour le cinquième aspect principal de l'invention, un distributeur selon l'invention peut encore comporter une ou plusieurs des caractéristiques optionnelles et préférées suivantes :
- le clapet anti-retour comporte un rebord, de préférence annulaire, pris en sandwich entre le réservoir, de préférence une extrémité distale du réservoir, de préférence une extrémité distale d'une canule du réservoir débouchant dans la cuve, et un embout d'application rigidement solidaire du réservoir, de préférence de manière que le rebord établisse une étanchéité entre ladite extrémité distale et l'embout d'application ; le clapet anti-retour peut être ainsi utilisé pour éviter tout passage de produit entre l'embout d'application et le réservoir, et en particulier entre l'embout d'application et la canule du réservoir sur laquelle l'embout d'application est monté ;
- un conduit, de préférence défini par l'embout d'application, s'étend depuis ledit clapet anti-retour jusqu'à l'orifice d'éjection par lequel du produit initialement contenu dans le réservoir peut être extrait du distributeur ;
- le clapet anti-retour est un clapet à reprise de goutte, c'est-à-dire dont la fermeture crée une aspiration ramenant du produit contenu dans ledit conduit, c'est-à-dire en aval du clapet anti-retour, du côté du clapet anti-retour opposé audit conduit, c'est-à-dire en amont du clapet anti-retour ;
- le capot comporte un téton disposé de manière à pénétrer dans ledit conduit dans la position fermée du capot, de préférence de manière à obturer, de préférence de manière étanche, ledit conduit ;
- le téton est configuré de manière à prendre appui sur le clapet anti-retour, de préférence de manière étanche, dans la position fermée du capot.

**Selon un sixième aspect principal de l'invention,** un distributeur selon l'invention comporte une étiquette indicatrice fixée, par une extrémité proximale, sur le piston et présentant une extrémité distale libre disposée contre la surface extérieure de la cuve.

Avantageusement, le déplacement du piston, selon l'axe X, dans le sens d'avancement, entraine celui de ladite extrémité distale de l'étiquette indicatrice, selon l'axe X, dans le sens de recul opposé au sens d'avancement. Le déplacement de l'étiquette indicatrice est en proportion de l'avancement du piston, et donc du nombre de doses délivrées. L'étiquette indicatrice constitue ainsi un moyen simple et fiable pour indiquer, par exemple, le nombre de doses déjà délivrées ou restant à délivrer.

Notamment pour le sixième aspect de l'invention, un distributeur selon l'invention peut encore comporter une ou plusieurs des caractéristiques optionnelles suivantes :
- le distributeur comporte une coque dans laquelle est logée la cuve du réservoir et l'extrémité distale de l'étiquette indicatrice est disposée entre la cuve et la coque ;
- la coque, de préférence opaque, ménage une fenêtre à travers laquelle une portion de l'étiquette indicatrice est visible depuis l'extérieur de la coque ;
- l'étiquette indicatrice porte des indications, le pas entre deux indications successives, le long de l'axe X, étant constant et de préférence égal à la course de dosage, c'est-à-dire à la distance maximale que le cliquet peut parcourir le long de l'axe X dans le sens d'avancement ;
- la fenêtre est dimensionnée de manière qu'une ou deux indications, de préférence une seule indication soit visible ;
- les indications sont des nombres qui augmentent ou décroissent, le long de l'axe X, l'évolution entre deux nombres adjacents étant d'une unité ;
- les indications sont disposées de manière que le nombre apparaissant dans la fenêtre soit égal au nombre de doses déjà distribuées ou au nombre de doses restant à distribue ;
- l'extrémité distale de l'étiquette indicatrice est fixée à une extrémité distale de la tige de piston ;
- l'étiquette indicatrice est en appui sur une portion rectiligne d'un rebord d'une ouverture proximale de la cuve, ouverture par laquelle la tête de piston est introduite dans la cuve ;
- la portion rectiligne s'étend dans un plan perpendiculaire à l'axe X ;
- l'étiquette indicatrice est en appui sur le chant d'au moins une, de préférence plusieurs nervures de support faisant saillie de la surface extérieure de la cuve et s'étendant de préférence dans un plan parallèle à l'axe X ;
- le chant de ladite nervure de support, de préférence sensiblement rectiligne, s'étend depuis ladite portion rectiligne du rebord de la cuve et de préférence jusqu'à la surface extérieure de la cuve ;
- la longueur des nervures de support est de préférence supérieure à 0,3 fois, de préférence supérieure à 0,4 fois la longueur de la partie de l'étiquette indicatrice qui, dans la configuration initiale du distributeur, c'est-à-dire avant distribution de la première dose, s'étend le long de la cuve, à l'extérieur de la cuve.

**De manière générale,** l'invention peut comporter une ou plusieurs des caractéristiques optionnelles suivantes :
- la cuve contient un produit liquide ou pâteux, de préférence un produit vétérinaire, de préférence encore un produit antiparasitaire ;
- le distributeur comporte un embout d'application comportant une extrémité distale atraumatique, de préférence sensiblement ovoïde, voire sphérique, ou à arrêtes chanfreinées, destinée à entrer en contact avec la peau d'un animal à traiter ;
- le distributeur comporte un embout d'application amovible ;
- le capot est conformé de manière que le distributeur soit stable, c'est-à-dire qu'il ne bascule pas lorsqu'il repose "tête en bas", c'est-à-dire exclusivement en appui sur le capot dans la position fermée, sur une surface plane inclinée de 10°, de préférence de 15°, de préférence de 20° avec un plan horizontal, quelle que soit la position du piston ;
- le capot présente une surface frontale dimensionnée pour que l'axe vertical passant par le barycentre du distributeur traverse ladite surface frontale lorsque le distributeur est posé tête en bas sur un plan incliné de 5°, de préférence de 10°, de préférence de 15° de préférence de 20°, par rapport à un plan horizontal ;
- le cliquet est logé dans la coque et le distributeur comporte un bouton-poussoir faisant saillie hors de la coque et monté mobile, selon l'axe X par rapport à la coque, le bouton-poussoir étant couplé mécaniquement avec le cliquet de manière qu'une pression sur ledit bouton-poussoir dans le sens d'avancement fasse avancer le cliquet dans le sens d'avancement ;
- le distributeur ne comporte que :
   - la coque,
   - le réservoir,
   - le piston,
   - de préférence une étiquette indicatrice,
   - un curseur, de préférence monobloc, constitué par le cliquet et le ressort, et de préférence des bras de butée,
   - de préférence un embout d'application et
   - de préférence un bouton-poussoir.

Bien entendu, les caractéristiques décrites ci-dessus, et en particulier les caractéristiques d'un distributeur selon l'un quelconque des aspects principaux de l'invention, peuvent être combinées.

L'invention concerne encore un procédé de fabrication d'un distributeur selon le quatrième aspect principal de l'invention, ce procédé comportant les étapes successives suivantes :
a) assemblage des différentes pièces constitutives dudit distributeur et remplissage de la cuve avec un produit liquide, de préférence un produit vétérinaire;
b) désactivation de l'organe de blocage ;
c) conditionnement dudit distributeur dans un emballage, de préférence un emballage hermétique.

Avantageusement, un procédé de fabrication selon l'invention permet d'obtenir un distributeur conditionné prêt à être utilisé, sans nécessité de désactiver l'organe de blocage.

De préférence, à l'étape b), on avance le piston dans la cuve, de manière à remplir, au moins partiellement, de préférence complètement, l'embout d'application avec dudit produit liquide. Le distributeur est ainsi « amorcé », de sorte que, dès que l'acheteur a retiré le distributeur de son emballage, il peut immédiatement l'utiliser pour distribuer une dose précise.

De préférence, l'amorçage et la désactivation de l'organe de blocage résultent d'une même pression sur un bouton-poussoir mécaniquement couplé au cliquet du distributeur.

L'invention concerne enfin un distributeur fabriqué suivant un procédé selon l'invention. En particulier, l'invention concerne un distributeur selon l'invention conditionné dans un emballage, de préférence hermétique, et de préférence comportant un organe de blocage ayant été désactivé.

### Définitions

Par "liquide", on entend un produit qui n'est pas solide, ni gazeux. Ce terme inclut donc des produits pâteux.

Un plan « longitudinal » est un plan parallèle à l'axe X.

Un plan "radial" est un plan qui inclut l'axe X.

Un plan « transversal » est un plan perpendiculaire à l'axe X.

Pour la description détaillée, dans un souci de clarté, on appelle "plan horizontal" un plan parallèle au plan dans lequel s'étend le curseur. Le plan horizontal "médian" est le plan horizontal qui contient l'axe X.

Un « plan vertical » est un plan perpendiculaire à un plan horizontal. Un plan vertical "longitudinal" est un plan vertical parallèle à l'axe X. Le plan vertical longitudinal "médian" est le plan vertical longitudinal qui inclut l'axe X.

"Distal" et "proximal" sont utilisés pour qualifier des positions relatives de pièces ou de parties de pièces le long de l'axe X, une pièce ou une partie de pièce "distale" étant plus proche de l'extrémité du distributeur du côté de l'embout d'application du produit qu'une pièce ou une partie de pièce "proximale".

Le produit s'écoulant vers l'extrémité distale du distributeur, des positions « distale » et « proximale » peuvent être également qualifiées comme étant « en aval » et « en amont », respectivement.

Une "nervure" est une fine saillie, en forme de lame, dont l'épaisseur est de préférence inférieure à 2, 3, 5 ou 10 fois la longueur. De préférence, la hauteur d'une nervure est supérieure à son épaisseur, voire supérieure à 2, 3, 5, voire 10 fois son épaisseur.

Sauf indication contraire, les verbes "comprendre", "comporter" ou "présenter" doivent être interprétés de manière large, c'est-à-dire de manière non exclusive.

### Brève descriptions des figures

D'autres caractéristiques et avantages de l'invention apparaitront encore à la lecture de la description détaillée qui va suivre, et à l'examen du dessin annexé dans lequel :

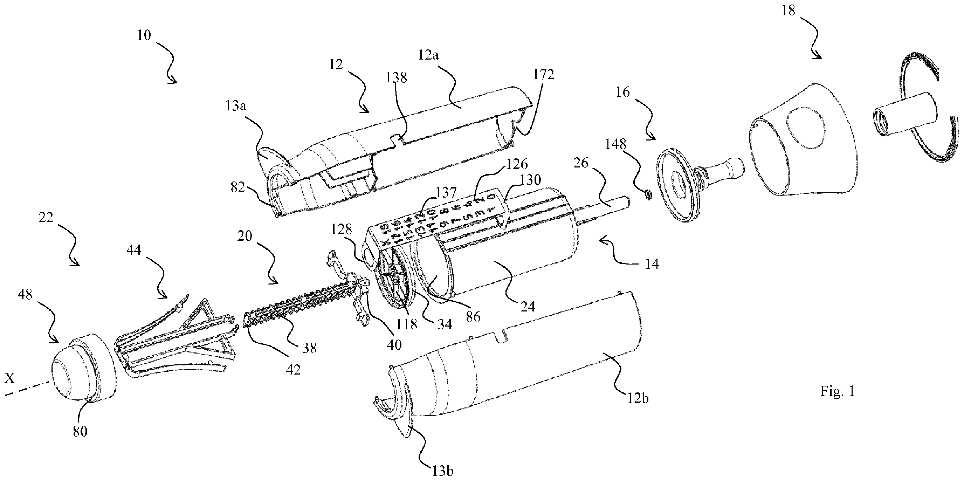

- la figure 1 représente, en vue éclatée, un distributeur selon un mode de réalisation préféré de l'invention ;
- la figure 2 (figures 2a - 2h) représente le distributeur de la figure 1 après assemblage, les figures 2a, 2d et 2h représentant le distributeur selon les coupes verticale médiane E-E, horizontale médiane B-B et transversale F-F, respectivement ;
- la figure 3 représente, en perspective, différentes pièces du distributeur des figures 1 et 2, les figures 3a et 3b représentant les demi-coques, la figure 3c représentant le curseur, les figures 3d et 3e représentant l'ensemble monobloc comportant la tige de piston et les pattes, les figures 3f et 3g représentant l'embout d'application, la figure 3h représentant le réservoir et les figures 3i et 3j représentant les deux pièces constitutives du capot, avant assemblage ;
- la figure 4 est une vue, en coupe transversale, des détails du distributeur des figures 1 et 2, les figures 4a, 4b et 4c illustrant le guidage de la tige de piston, le clapet anti-retour et les dents, respectivement.

Des références identiques sont utilisées pour désigner des organes analogues ou identiques. Un indice permet de distinguer ces organes. La référence sans indice est cependant utilisée pour désigner, de manière générique, un ensemble d'organes analogues ou identiques.

Le plan vertical longitudinal médian sépare l'espace en deux demi-espaces. Des références peuvent être affectées d'un signe "a" ou "b" selon qu'elles font référence à des pièces ou à des parties de pièces qui s'étendent dans le premier ou le deuxième demi-espace, respectivement.

### Description détaillée

### Structure générale

Les figures représentent un mode de réalisation préféré d'un distributeur 10 selon l'invention. Ce distributeur comporte une coque 12, un réservoir 14, un embout d'application 16, un capot 18 amovible, un piston 20 portant une crémaillère, et un poussoir 22 comportant un cliquet coopérant avec la crémaillère pour faire avancer, sous l'action d'un opérateur et de manière irréversible, le piston 20 dans le réservoir 14, de manière à pousser le produit contenu dans le réservoir vers l'embout d'application 16.

La coque 12 comporte deux demi-coques 12a et 12b fixées l'une à l'autre, par exemple par clipsage, collage ou soudure. Ces deux demi-coques définissent un volume intérieur sensiblement tubulaire, d'axe X, dans lequel sont notamment logés le réservoir 14 et le piston 20.

La coque 12 comporte des oreilles 13a et 13b, de préférence disposées à proximité de l'extrémité proximale de la coque 12, et de préférence venues de matière avec les demi-coques 12a et 12b, respectivement. Les oreilles 13a et 13b sont conformées de manière à servir de surface d'appui pour l'index et le majeur d'une main de l'opérateur, respectivement, afin de retenir la coque 12 lorsque l'opérateur appuie, avec son pouce, sur le poussoir 22.

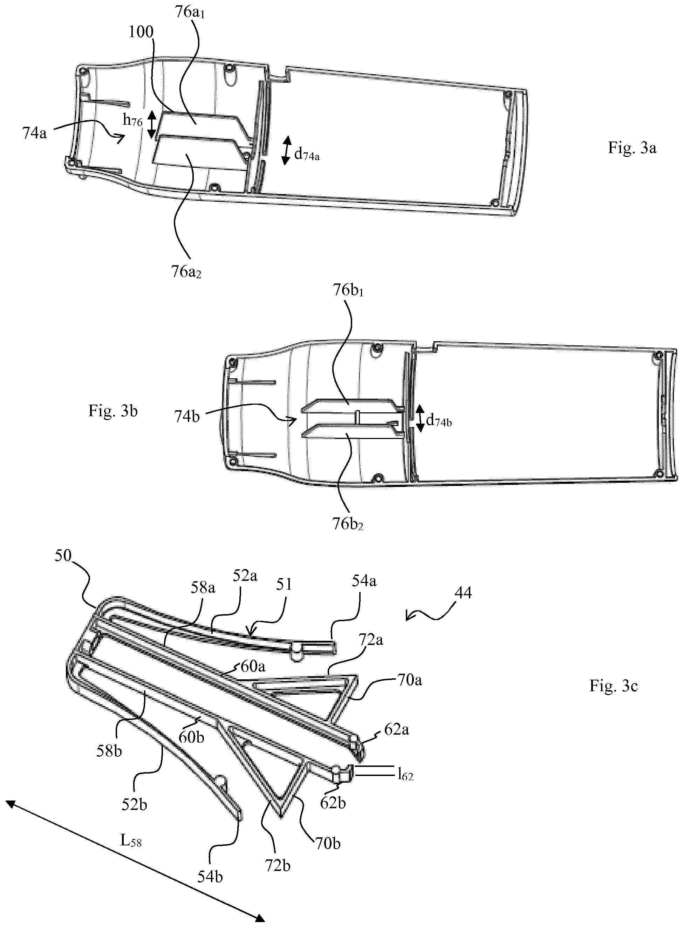

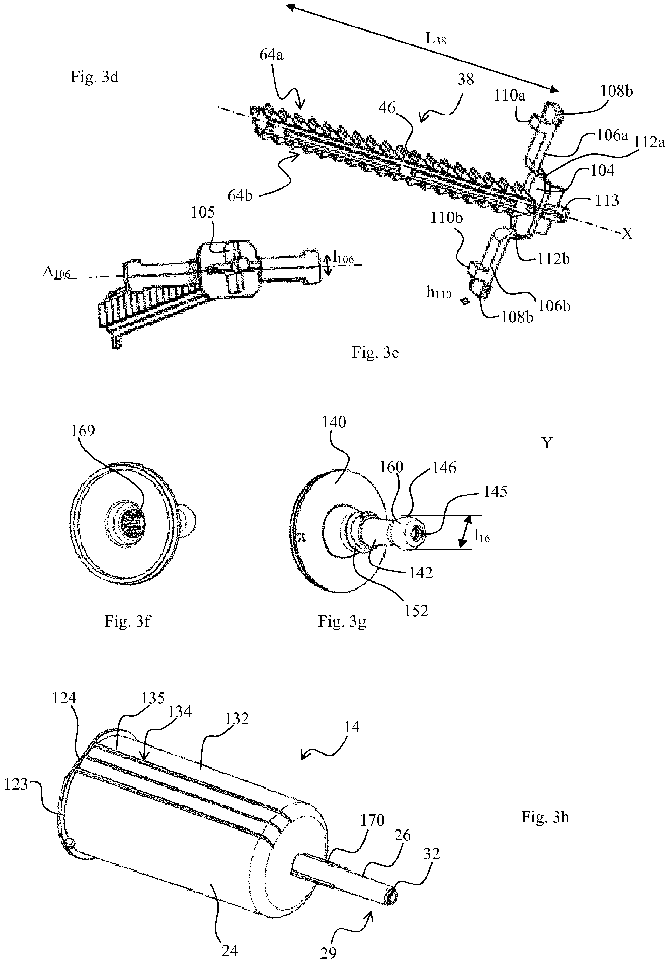

Le réservoir 14 comporte une cuve 24 et une canule 26. La cuve 24, d'axe X, présente une section constante, de préférence circulaire. La lumière 28 de la canule 26 débouche dans le fond de la cuve 24 et, à l'extrémité distale 29 opposée, par un orifice de sortie 32 (figure 3h).

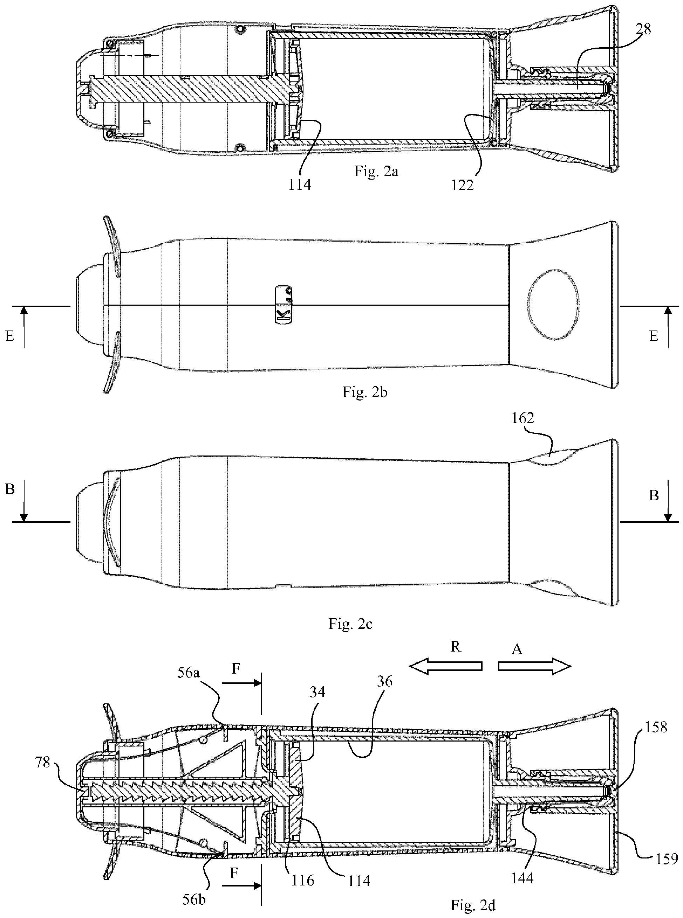

Le piston 20 comporte une tête de piston 34 configurée pour coulisser dans la cuve 24, selon l'axe X, en maintenant un contact étanche entre la surface intérieure 36 de la cuve et la tête de piston 34 (figure 2d).

Le piston 20 comporte encore une tige de piston 38 dont une extrémité distale 40 est fixée sur la tête de piston 34, et une extrémité proximale 42, opposée à l'extrémité distale 40, est libre.

Le poussoir 22 comporte un curseur 44 mobile en translation selon l'axe X et coopérant avec une crémaillère, en l'occurrence un ensemble de dents 46 ménagées sur la tige de piston 38, de manière que la tige de piston 38 ne suive le mouvement du curseur 44 que lorsque ce dernier se déplace vers la canule 26 (sens d'avancement A). Le poussoir comporte également un bouton-poussoir 48 couplé avec le curseur 44 et actionnable, depuis l'extérieur de la coque 12, par un opérateur.

### Curseur

Le curseur 44 (figure 3c) s'étend dans le plan horizontal médian. Il comporte, à son extrémité distale, une base 50 sensiblement transversale, c'est-à-dire s'étendant dans un plan perpendiculaire à l'axe X.

Le curseur comporte un ressort 51, sous la forme de deux branches de ressort 52a et 52b qui, agissant comme des lames ressort, s'étendent, à partir de la base 50, vers l'extrémité distale du distributeur, dans le plan horizontal médian. Chaque branche de ressort 52a et 52b diverge progressivement de manière à s'écarter de l'axe X, jusqu'à une extrémité d'appui 54a et 54b, respectivement en appui sur des épaulements 56a et 56b définis par la coque 12 (figure 2d). L'appui des extrémités d'appui 54a et 54b sur les épaulements 56a et 56b empêche avantageusement la rotation du curseur 44 autour d'un axe vertical perpendiculaire au plan horizontal médian.

Les branches de ressort s'effilent progressivement à mesure que l'on s'approche des extrémités d'appui, au moins sur une partie de leur longueur.

La forme des branches de ressort est adaptée pour autoriser leur déformation, dans le plan horizontal médian, lorsqu'une force est exercée au moyen du bouton-poussoir 48 sur la base 50, selon l'axe X et vers l'extrémité distale du distributeur. Sous l'effet de cette déformation, la courbure des branches de ressort augmente. Lorsque cette force disparaît, l'élasticité des branches de ressort leur permet de reprendre leur forme initiale.

Le curseur 44 comporte encore deux branches de cliquet 58a et 58b s'étendant, sensiblement selon l'axe X, entre les branches de ressort 52a et 52b.

Les branches de ressort et les branches de cliquet s'étendent sensiblement dans le même plan. Les branches de cliquet sont écartées l'une de l'autre de manière que la tige de piston 38 puisse se loger entre lesdites branches de cliquet.

Plus précisément, chaque branche de cliquet 58a - 58b (figure 3c) comporte :
- une partie longitudinale 60a-60b qui, depuis le base 50, s'étend, selon l'axe X, vers l'extrémité distale du distributeur, au-delà des extrémités d'appui des branches de ressort, et
- une partie de pince 62a - 62b, qui prolonge la partie longitudinale 60a-60b et vient en contact avec la tige de piston.

Les deux branches de cliquet et la base 50 présentent ainsi la forme générale d'une pince, similaire à une pince à épiler, venant pincer élastiquement la tige de piston 38. L'action de pincement se traduit par l'application, par les parties de pince, de deux forces opposées, orientées vers le plan horizontal médian.

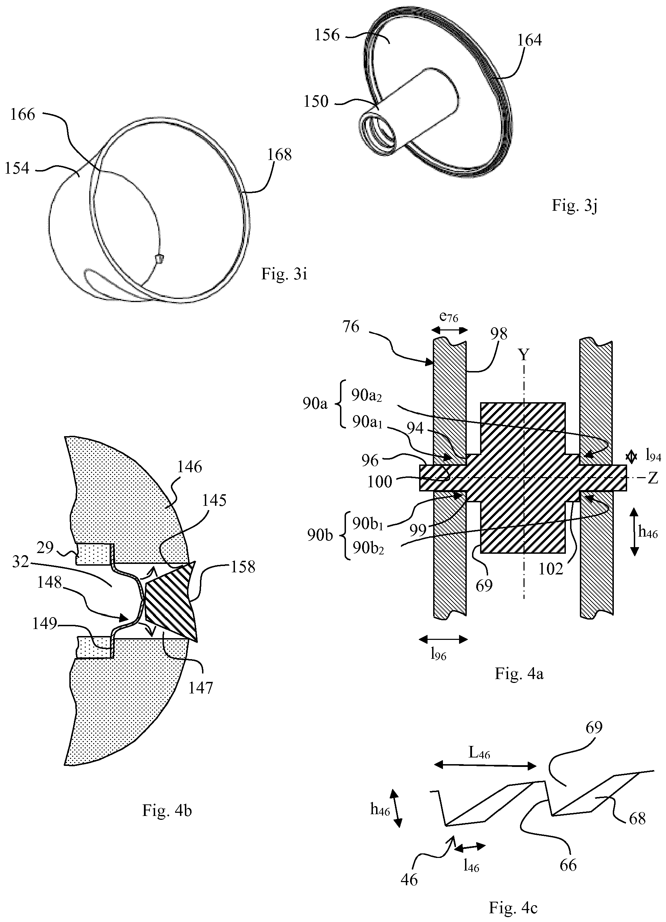

Dans la région de pincement, la tige de piston 38 présente une crémaillère formée, sur deux faces opposées de la tige de piston, par des rangées de dents 64a et 64b (figure 3d). Les rangées de dents 64a et 64b coopèrent avec les parties de pince 62a et 62b, respectivement, pour assurer un avancement irréversible du piston. A cet effet, chaque dent 46 comporte une face de blocage 66 sensiblement transversale et sensiblement rectangulaire, une face inclinée 68 qui, depuis la face de blocage, se rapproche de l'axe X à mesure que l'on se rapproche de l'extrémité distale du distributeur, et deux faces latérales 69 verticales (figure 4c). La face de blocage 66 d'une dent d'une rangée de dents 64a ou 64b est conformée de manière à bloquer l'avancement de la partie de pince 62a ou 62b, respectivement, dans le sens d'avancement A. Au contraire, la face inclinée 68 d'une dent quelconque d'une rangée de dents 64a ou 64b est conformée de manière à autoriser un contact glissant de la partie de pince 62a ou 62b, respectivement, vers l'extrémité proximale du distributeur.

De préférence, toutes les dents 46 d'une rangée de dents sont identiques, de préférence toutes les dents des deux rangées de dents sont identiques. De préférence, la hauteur h₄₆ d'une dent 46 est supérieure à 0,1 mm, de préférence supérieure à 0,2 mm, de préférence supérieure à 0,5 mm, voire supérieure à 1 mm et/ou inférieure à 5 mm, de préférence inférieure à 3 mm. De préférence, la longueur L₄₆ d'une dent 46 est supérieure à 1 mm, de préférence supérieure à 2 mm et/ou inférieure à 6 mm, de préférence inférieure à 5 mm, respectivement.

De préférence encore, la largeur l₄₆ d'une dent 46 est supérieure à 1 mm et/ou inférieure à 8 mm. De préférence, elle est sensiblement identique à la largeur l₆₂ de la partie de pince avec laquelle la dent 46 coopère.

Le nombre de dents n'est pas limité. De préférence, le nombre de dents d'une rangée de dents est supérieur à 10, supérieur à 15 et/ou inférieur à 30, inférieur à 25, ou inférieur à 20.

Le curseur 44 comporte encore des bras de butée 70a et 70b destinés à limiter la course d'avancement du curseur selon l'axe X. De préférence, les bras de butée 70a et 70b sont fixés sur les branches de cliquet 58a et 58b, respectivement. De préférence encore, ils s'étendent sensiblement transversalement. Dans un mode de réalisation, les deux extrémités de chaque bras de butée sont connectées à une même branche de cliquet, une desdites extrémités étant reliée par l'intermédiaire d'un bras de rigidification 72a-72b.

De préférence, les bras de butée et les bras de rigidification s'étendent dans le même plan que les branches de cliquet et, de préférence encore, dans le même plan que les branches de ressort. De préférence encore, les largeurs, mesurées perpendiculairement au plan général du curseur 44, de la base 50 et/ou des branches de ressort 52a-52b et/ou des branches de cliquet 58a-58b et/ou des bras de butée 70a-70b et/ou des bras de rigidification 72a-72b sont identiques.

Le distributeur comporte des moyens pour guider les mouvements d'avancement, c'est-à-dire selon le sens d'avancement A, et de recul, suivant le sens opposé, dit "sens de recul" R, du curseur 44. De préférence, ces moyens de guidage sont conformés de manière à n'autoriser qu'une translation selon l'axe X.

A cet effet, le distributeur comporte deux couples 74a et 74b de nervures de guidage 76ai et 76a₂, et 76b₁ et 76b₂, respectivement. De préférence, les couples de nervures de guidage 74a et 74b sont venus de matière avec la coque 12 et, de préférence avec les demi-coques 12a et 12b, respectivement. La distance d₇₄ₐ ou d_{74b} entre les nervures de guidage 76a₁ et 76a₂ ou 76b₁ et 76b₂, respectivement, correspond sensiblement à la largeur des bras de butée et/ou des bras de rigidification et/ou des branches de cliquet et/ou des branches de ressort, en étant légèrement supérieure à cette largeur de manière à autoriser un coulissement guidé du curseur entre lesdites nervures de guidage. De préférence, l'épaisseur des différentes parties du curseur 44 susceptibles de pénétrer entre les nervures de guidage d'un couple de nervures de guidage lors du mouvement de va-et-vient du curseur 44 est constante, ce qui permet d'améliorer la qualité du guidage.

De préférence, la base 50 est en appui sur le fond du bouton-poussoir 48. De préférence encore, le curseur 44 est fixé sur le fond du bouton-poussoir 48, par exemple par insertion d'un tenon 78 du bouton-poussoir dans un logement correspondant de la base 50 du curseur 44.

Dans un mode de réalisation préféré, le bouton-poussoir 48 est en un matériau rigide. Il présente de préférence un rebord périphérique 80 qui, en coopération avec un rebord annulaire 82 de la coque 12, limite le mouvement de recul du bouton-poussoir 48.

De préférence encore, le bouton-poussoir 48 est creux, ce qui avantageusement limite l'encombrement axial du distributeur, les branches de cliquet 58a et 58b et les branches de ressort 52a et 52b pouvant s'étendre sensiblement jusqu'au fond du bouton-poussoir 48.

Comme décrit ci-dessus, le curseur 44 permet de transmettre un mouvement d'avancement à la tige de piston 38 par coopération des parties de pince 62a et 62b avec les dents des rangées de dents 64a et 64b respectivement.

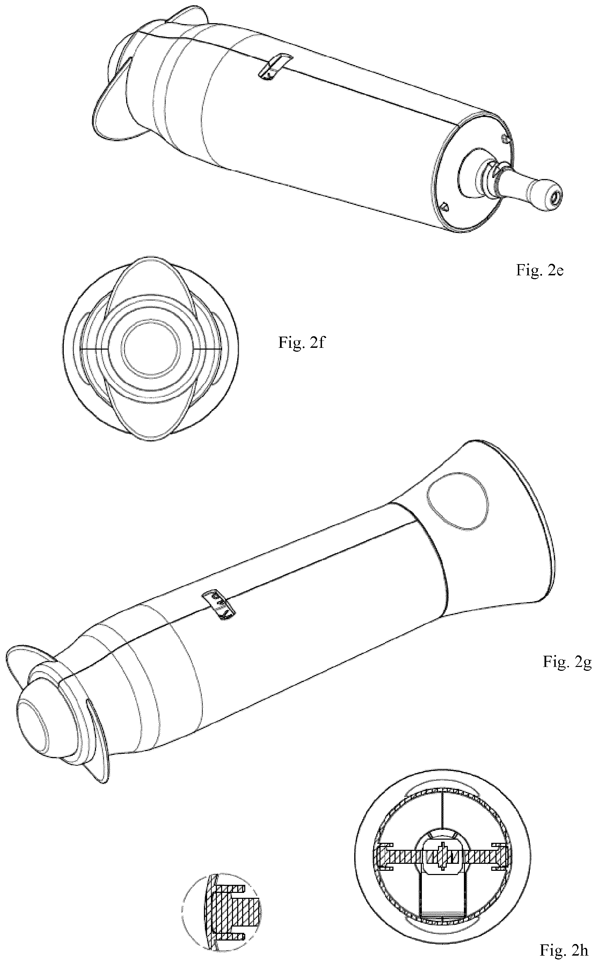

La position de recul maximal du piston 20 correspond de préférence à la configuration initiale du distributeur, avant sa première utilisation (figure 2). Dans la configuration initiale, les rangées de dents 64a et 64b font face aux branches de cliquet 58a et 58b, respectivement et, de préférence, la tête de piston 34 obture l'ouverture proximale 86 de la cuve 24.

De préférence encore, la longueur L₅₈ des branches de cliquet est sensiblement égale à la longueur de la tige de piston L₃₈. Dans la position de recul maximal du piston 20, comme représenté sur la figure 2d, la tige de piston 38 peut être avantageusement logée entre les deux branches de cliquet 58a et 58b, l'extrémité proximale 42 de la tige de piston 38 étant à proximité immédiate de la base 50. La compacité du distributeur en est améliorée.

Dans un mode de réalisation préféré, les rangées de dents 64a et 64b s'étendent sur toute la longueur de la tige de piston. La course d'avancement du piston 20 peut ainsi être avantageusement maximisée, puisqu'elle peut atteindre toute la longueur L₃₈ de la tige de piston.

Le mouvement d'avancement de la tige de piston est guidé par les nervures de guidage 76a₁, 76a₂, 76b₁ et 76b₂ afin que la tige de piston ne puisse que translater selon l'axe X, en excluant toute rotation. A cet effet, comme représenté sur la figure 4a, des premier et deuxième couples 90a et 90b de cornières 92a₁ et 92a₂, et 92b₁ et 92b₂, respectivement, sont ménagés le long de la tige de piston 38. Les cornières des premier et deuxième couples 90a et 90b s'étendent le long et de chaque côté des première et deuxième rangées de dents, respectivement.

Chaque cornière 92 définit une face verticale 94 et une face horizontale 96 qui sont en contact glissant avec une grande face, en l'occurrence une face intérieure 98, et un chant 100, respectivement, d'une nervure de guidage 76 correspondante, comme représenté sur la figure 4a.

Plus précisément, les chants des nervures de guidage 76a₁ et 76a₂, respectivement, sont coplanaires et prennent appui sur les faces horizontales coplanaires des cornières 92a₁ et 92a₂, respectivement.

De même, les chants des nervures de guidage 76b₁ et 76b₂, respectivement, sont coplanaires et prennent appui sur les faces horizontales coplanaires, des cornières 92b₁ et 92b₂ respectivement.

Les faces intérieures des nervures de guidage 76a₁ et 76a₂, respectivement, prennent appui sur les faces verticales des cornières 92b₁ et 92b₂, respectivement. Les faces intérieures des nervures de guidage 76b₁ et 76b₂, respectivement, prennent appui sur les faces verticales des cornières 92b₁ et 92b₂, respectivement.

Les faces verticales des cornières 90a₁ et 90b₁ sont coplanaires, comme les faces verticales des cornières 90a₂ et 90b₂.

Pour améliorer la qualité du guidage, chaque nervure de guidage 76 présente de préférence un chant 100 qui s'étend parallèlement à l'axe X. La longueur du chant 100 est de préférence supérieure à 5 mm, de préférence supérieure à 10 mm.

La hauteur h₇₆ d'une nervure de guidage, mesurée depuis la coque jusqu'au chant 100, est de préférence la même, quelle que soit la nervure de guidage considérée.

Cet agencement limite efficacement toute rotation de la tige de piston autour de l'axe X.

Les coûts de fabrication en sont réduits. La compacité du distributeur est également améliorée.

La présence des quatre nervures de guidage, coopérant avec quatre cornières respectives de la tige de piston 38, assure un guidage précis et fiable de la tige de piston 38.

De préférence, l'épaisseur e₇₆ d'une nervure de guidage est supérieure 0,3 mm et/ou inférieure à 2 mm. De préférence, l'épaisseur e₇₆ est sensiblement égale à la largeur l₉₆ de la face horizontale 96 de la cornière 94 avec laquelle la nervure de guidage 76 coopère. Dans un mode de réalisation préféré, la largeur l₉₆ d'une face horizontale 96 est supérieure à 0,3 mm et/ou inférieure à 2 mm.

Dans un mode de réalisation préféré, la largeur l₉₄ de la face verticale 94 d'une cornière est inférieure à 2 mm, de préférence inférieure à 1 mm. De préférence, la largeur l₉₄ de la face verticale d'une cornière 92 est inférieure à la hauteur h₄₆ des dents de la rangée de dents le long de laquelle ladite cornière s'étend.

De préférence encore, les quatre cornières sont sensiblement identiques et/ou les quatre nervures de guidage sont sensiblement identiques.

De préférence, dans une section transversale quelconque, comme représenté sur la figure 4a, la section de la tige de piston présente deux axes de symétrie Y et Z. De préférence, elle présence la forme générale d'une croix quand elle coupe une dent.

De préférence, la face verticale 94 d'une cornière ne s'étend pas dans le plan d'une face latérale 69 d'une dent. Dans un mode de réalisation préféré, un rebord 102, de préférence sensiblement horizontal, relie la face verticale 94 d'une cornière à la face latérale 69 des dents de la rangée de dents qui s'étend le long de ladite cornière. L'arête 99 délimitant la face verticale 94, du côté opposé à la ligne de jonction entre la face verticale 94 et la face horizontale 96 d'une cornière, est de préférence rectiligne et s'étend de préférence parallèlement à l'axe X. La fiabilité du guidage en est améliorée.

L'extrémité distale 40 de la tige de piston 38 définit un plateau 104, transversal, qui prend appui sur la tête de piston 34. Avantageusement, cet appui évite toute rotation de la tête de piston autour d'un axe différent de l'axe X. De préférence, le plateau 104 se prolonge par une ou plusieurs lamelles 105 qui pénètrent dans la tête de piston, et évitent ainsi toute rotation de la tête de piston autour de l'axe X. Les lamelles 105 s'étendent de préférence dans des plans longitudinaux, de préférence radiaux.

Réciproquement, le plateau 104 permet à la tige de piston 38 de bénéficier du guidage de la tête de piston 34 assuré par la cuve 24.

### Organe de blocage

Le distributeur comporte un organe de blocage, sous la forme de deux pattes 106a et 106b (figure 3e). Les pattes s'étendent dans un plan transversal.

De préférence, les pattes sont à l'intérieur de la coque, et ne sont pas accessibles par l'opérateur. La sécurité en est améliorée.

Les pattes sont alignées selon un axe de pattes Δ₁₀₆ transversal, qui coupe l'axe X.

Les pattes 106a et 106b sont fixées sur le plateau 104 par des extrémités radialement intérieures 112a et 112b, respectivement. La zone de jonction entre une extrémité intérieure 112 et le plateau 104 est une zone d'affaiblissement, sécable sous l'effet d'une pression exercée sur la tige de piston 38, dans le sens d'avancement, notamment sous l'effet d'une pression de l'opérateur sur le bouton-poussoir, par exemple au moyen de son pouce.

La zone d'affaiblissement a la forme d'une ligne d'affaiblissement, de préférence perpendiculaire à l'axe X, par exemple s'étendant sensiblement verticalement. La ligne d'affaiblissement peut résulter d'une réduction locale d'épaisseur ou de la présence de trous traversant.

Chaque patte 106a et 106b comporte une extrémité radialement extérieure 108a et 108b, respectivement, fixée sur la coque 12, de préférence sur les demi-coques 12a et 12b, respectivement. De préférence, chaque extrémité radialement extérieure 108a et 108b est fixée par insertion dans un logement correspondant ménagé sur la coque, en particulier entre les couples de nervures 76a₁-76a₂, et 76b₁-76b₂, respectivement. De préférence, la fixation est obtenue par un encliquetage.

Dans un mode de réalisation préféré, les pattes 106a et 106b s'étendent au moins partiellement entre les nervures de guidage des couples 74a et 74b, respectivement. De préférence, la largeur l₁₀₆ des pattes 106a et 106b (en dehors de leurs parties d'extrémité ; voir figure 3e), est sensiblement égale à la distance entre les deux nervures de guidage entre lesquelles elles s'étendent. Avantageusement, les nervures de guidage contribuent donc au maintien en position des pattes, ce qui est en particulier utile après rupture de la zone de jonction.

De préférence, les pattes 106a et 106b servent de butée aux bras de butée 70a et 70b, respectivement, pour limiter l'avancement du curseur 44.

A cet effet, de préférence, les pattes 106a et 106b comportent chacune, de préférence à proximité de leurs extrémités radialement extérieures, un pion de butée 110a et 110b, respectivement.

A chaque pression sur le bouton-poussoir pour délivrer une dose, le curseur 44 avance depuis sa position de repos dans laquelle le rebord périphérique 80 du bouton-poussoir est en butée sur la coque jusqu'à la position d'avancement maximal dans laquelle les bras de butée 70 sont en butée sur les pions de butée. Le curseur entraîne le piston. La hauteur h₁₁₀ des pions de butée détermine donc la course de dosage, c'est-à-dire l'avancement du piston 20 à chaque pression sur le bouton- poussoir.

De préférence, la hauteur h₁₁₀ est déterminée pour que la course de dosage soit égale à la longueur L₄₆ d'une dent. A chaque actionnement du bouton-poussoir, le piston avance ainsi, selon l'axe X, sur une distance L₄₆.

De préférence, la tige de piston 38 (qui comprend le plateau 104) et les pattes 106 constituent une pièce monobloc, par exemple fabriquée par injection de plastique dans un moule.

Avantageusement, la hauteur des pions de butée 110 peut alors déterminer une course de dosage nécessairement égale à la longueur d'une ou plusieurs dents L₄₆, ce qui optimise la précision du dosage

Cette caractéristique est particulièrement avantageuse pour modifier le volume de chaque dose distribuée par le distributeur. Il suffit en effet de changer la pièce monobloc en la remplaçant par une autre pièce analogue, mais présentant par exemple, pour une course de dosage accrue, une longueur de dents plus longue et des pions de butée moins haut.

Les pions de butée étant initialement fixés sur le piston portant la crémaillère, l'opérateur ne risque pas d'utiliser une crémaillère et des pions de butée qui ne correspondent pas. L'assemblage du distributeur en est facilité et est plus sûr.

### Piston

L'extrémité distale 40 de la tige de piston 34 comporte un tenon de fixation 113 d'axe X introduit et fixé dans un logement correspondant de la tête de piston 34, sensiblement au centre de la tête de piston.

La tête de piston 34, d'axe X, comporte un support de joint 114 de forme générale discoïdale, et un joint 116 de préférence logé dans une gorge circonférentielle du support de joint 114 de manière à assurer une étanchéité entre la surface intérieure 36 de la cuve et la tête de piston 34 (figure 2d).

Dans un mode de réalisation préféré, le support de joint 114 comporte des nervures de rigidification 118, de préférence sensiblement radiales, permettant avantageusement de limiter le poids du support de joint et la quantité de matière utilisée pour le réaliser.

La face frontale 120 du support de joint 114 est de préférence de forme complémentaire au fond 122 de la cuve 24 dans lequel débouche la lumière de canule 28. De préférence, la longueur de la tige de piston 38 est suffisante pour que la face frontale du support de joint 114 puisse atteindre le fond 122 de la cuve 24. La longueur de la course d'avancement de la tête de piston dans la cuve 24 est de préférence sensiblement identique à la longueur de la tige de piston 38. Avantageusement, la cuve 24 peut être ainsi totalement vidée, ce qui limite les pertes de produit.

De préférence, la longueur de la tige de piston est déterminée pour que, dans cette position extrême du piston 20, correspondant à une "configuration finale", les parties de pince 62 du curseur 44 soient en amont des faces de blocage 66 des « dernières » dents 46, c'est-à-dire situées à l'extrémité proximale 42 de la tige de piston 38. La compacité du distributeur est ainsi optimale.

### Cuve

La cuve 24, d'axe X, présente une section constante le long de l'axe X, de préférence circulaire. Son ouverture proximale 86 est bordée par un rebord 123, qui s'étend dans un plan sensiblement transversal et présente une forme générale annulaire. Le rebord 123 présente cependant une portion rectiligne, sous la forme d'un méplat 124, sensiblement horizontal. Trois nervures de support 134, longitudinales, sont venues de matière avec la cuve. Leur chant 135, rectiligne, s'étend depuis la surface extérieure 132 de la cuve jusqu'au méplat 124.

### Etiquette indicatrice

Une étiquette indicatrice 126 est fixée par une extrémité proximale 128 sur le piston 20. Par exemple, dans le mode de réalisation représenté, l'extrémité proximale 128 comporte un orifice qui est traversé par le tenon de fixation 113 de manière que ladite extrémité proximale 128 soit prise en sandwich entre le plateau 104 et le support de joint 114. L'extrémité distale 130 de l'étiquette indicatrice est libre. L'étiquette indicatrice 126 s'étend, sensiblement selon l'axe X, de préférence sensiblement à plat, entre la coque 12 et la surface extérieure 132 de la cuve 24, en s'appuyant sur le méplat 124 et sur les nervures du support 134.

Sur sa face extérieure, l'étiquette indicatrice porte des indications 137, par exemple des chiffres et/ou des couleurs, de préférence une suite ordonnée de chiffres qui augmentent ou diminuent à mesure que l'on s'approche de l'extrémité distale 130 de l'étiquette indicatrice 126. Une fenêtre 138, ménagée dans la coque 12, est configurée pour que l'opérateur puisse apercevoir au moins une indication 137, de préférence seulement une indication 137 à travers la fenêtre 138.

Le pas, c'est-à-dire l'écartement selon l'axe X entre deux indications 137 successives, est sensiblement égal à la course de dosage. Avantageusement, l'indication 137 visible par la fenêtre 138 évolue donc à chaque fois qu'une dose est délivrée. La lecture à travers la fenêtre 138 permet ainsi, par exemple, de visualiser un nombre correspondant au nombre de doses déjà délivrées ou au nombre de doses qu'il est encore possible de délivrer.

La fixation de l'extrémité proximale 128 de l'étiquette indicatrice sur le piston permet de tirer cette extrémité vers l'intérieur de la cuve à mesure que le piston avance dans ladite cuve. L'étiquette indicatrice est suffisamment souple pour pouvoir se courber à 180° pour contourner le rebord 123 de la cuve, de manière réversible. Elle peut être notamment en papier et/ou en plastique.

Les nervures du support 134 favorisent le déplacement de l'étiquette indicatrice 126 et sa rotation autour du méplat 124. Le méplat 124 permet également, avantageusement, de conférer une forme plate à l'étiquette indicatrice dans la région de la fenêtre 138.

### Embout d'application

L'embout d'application 16 est destiné à faciliter l'application du produit sur la peau d'un animal.

De préférence, l'embout d'application (figures 3f et 3g) est donc atraumatique, et en particulier, ne présente pas une forme pointue. De préférence, l'extrémité distale de l'embout d'application présente une largeur l₁₆, mesurée dans un plan transversal, supérieure à 1 mm, supérieure à 2 mm, ou supérieure à 3 mm. L'extrémité distale 146 de l'embout d'application présente de préférence une protubérance 160 dépourvue d'angles vifs, de préférence sensiblement sphérique, ce qui limite les risques de blessure pendant la distribution du produit. Le rayon de courbure de la protubérance est de préférence supérieur à 1 mm, à 2 mm ou à 3 mm.

L'embout d'application 16 représenté comporte une base annulaire 140 prolongée axialement, du côté proximal, par une tétine 142.

L'embout d'application peut être fixé sur la coque 12 pour tout moyen, par exemple par clipsage de la base annulaire 140 sur la coque. Dans un mode de réalisation, l'embout d'application 16 est monté de manière amovible sur la coque. Avantageusement, il peut être ainsi remplacé par un embout mieux adapté au produit à appliquer ou à l'animal sur lequel le produit doit être appliqué.

Une lumière d'embout 144 traverse de part en part, selon l'axe X, la base annulaire et la tétine 142 et débouche axialement vers l'extérieur par un orifice d'éjection 145, à l'extrémité distale 146 de la tétine 142. La canule 26 est logée dans la lumière d'embout 144 jusqu'à atteindre sensiblement l'extrémité distale 146 de la tétine.

Un clapet anti-retour 148 (figure 4b) est cependant pris en sandwich entre l'extrémité distale 29 de la canule 26 et l'extrémité distale 146 de la tétine (figure 4b). L'orifice de sortie 32 de la canule est donc axialement légèrement décalé, vers l'amont, par rapport à l'orifice d'éjection 145. Le conduit 147 qui s'étend entre l'orifice de sortie 32 et l'orifice d'éjection 145 présente de préférence une longueur inférieure à 1 mm.

Le clapet anti-retour obture l'orifice de sortie 32 de la canule. Il est configuré de manière à s'ouvrir sous l'effet d'une surpression dans la cuve (flèches sur la figure 4b), ce qui permet d'éjecter du produit initialement contenu dans la cuve, et de manière à rester fermé en l'absence d'une telle surpression, ce qui garantit des conditions d'hygiène optimales.

Le clapet anti-retour, par exemple en un matériau polymère, par exemple en caoutchouc, peut être en particulier une membrane flexible fendue, en forme de dôme. Comme représenté sur la figure 4b, le clapet anti-retour comporte un rebord 149 pris en sandwich et comprimé entre la tétine 142 et la canule 26. Avantageusement, le rebord 149, de préférence annulaire, assure une étanchéité entre la tétine et la canule, évitant toute fuite de produit entre la canule et la tétine.

Pour assurer un positionnement angulaire prédéterminé, autour de l'axe X, de l'embout d'application 16 et de la cuve sur la coque 12, la surface intérieure de la tétine, définissant la lumière 144, comporte une ou plusieurs rainures longitudinales 169 (figure 3f) dans lesquelles sont logées une ou plusieurs nervures de détrompage 170 disposées à la surface extérieure de la canule de la cuve.

Les mêmes nervures de détrompage 170 sont également logées dans des échancrures 172 de forme correspondante, ménagées dans la coque.

### Capot

Le capot 18 (figures 3i et 3j) comporte de préférence une jupe intérieure 150, intérieurement filetée de manière à pouvoir être vissée sur un filet de tétine 152, et une jupe extérieure 154, les jupes intérieure et extérieure étant reliées l'une à l'autre par une voûte transversale 156, en forme de disque.

De préférence, le capot 18 est constitué de deux pièces fixées l'une à l'autre, à savoir la jupe extérieure 154 d'une part et une pièce constituée de la jupe intérieure 150 et de la voûte transversale 156, ces deux pièces étant de préférence soudées l'une à l'autre à la périphérie de la voûte 156.

La longueur de la jupe intérieure 150 est déterminée de manière que, dans la position fermée du capot, correspondant à un vissage maximal du capot sur le filet de tétine 152, la voûte transversale 156 obture l'orifice d'éjection 145 de la tétine. De préférence, la voûte transversale comporte un téton 158 configuré de manière à obturer le clapet anti-retour 48 dans la position fermée du capot. De préférence, le téton 158 pénètre dans le conduit 147 (figure 4b), de préférence de manière à obturer de manière étanche l'orifice d'éjection 145, de préférence encore en prenant appui sur le clapet anti-retour de manière à empêcher son ouverture. Les conditions d'hygiène en sont améliorées, aucune goutte du produit ne pouvant sortir du distributeur dans la position fermée du capot 18.

L'obturation étanche de l'orifice d'éjection 145 par le capot, dans la position fermée, empêche également, avantageusement, tout déplacement du piston 20 dans le sens de recul R.

La surface frontale 159 du capot est de préférence plane. Elle présente de préférence une surface supérieure à 3 cm², à 5 cm², à 8 cm², permettant de poser le distributeur tête en bas. De préférence, les dimensions de la surface frontale 159 sont adaptées pour que l'axe vertical passant par le barycentre du distributeur traverse ladite surface frontale lorsque le distributeur est posé tête en bas sur un plan incliné de 5°, de préférence de 10°, de préférence de 15°, de préférence de 20°, par rapport à un plan horizontal. Posé tête en bas, c'est-à-dire avec ladite surface frontale 159 en contact avec ledit plan incliné, le distributeur est donc stable. Il est ainsi avantageusement facile à ranger.

La jupe extérieure 154 présente de préférence des moyens de verrouillage permettant de bloquer le capot 18 sur la coque 12 ou sur l'embout d'application 16. De préférence, ces moyens de verrouillage sont activés par le seul vissage du capot sur le filet de tétine 152. De préférence, ces moyens de verrouillage ne peuvent être déverrouillés par le seul dévissage du capot. De préférence, ils nécessitent une déformation de la jupe extérieure 154, de préférence par pression sur des régions de déverrouillage 162, de préférence ménagées en relief sur la jupe extérieure 154. Les moyens de verrouillage peuvent par exemple consister en des moyens de clipsage, bien connus.

Le vissage du capot sur le filet de tétine 152 amène la jupe extérieure 154 dans une position angulaire autour de l'axe X prédéfinie, déterminée pour que les moyens de verrouillage puissent être activés en fin de vissage et être désactivés, par pression sur les régions de déverrouillage 162, dans la position vissée.

Pour garantir un positionnement angulaire adapté des régions de déverrouillage 162 dans la position vissée du capot, la voûte transversale 156 présente de préférence un détrompeur 164, notamment sous la forme d'une surface plate venant se positionner précisément le long d'un bord rectiligne 166 correspondant du rebord distal 168 de la jupe extérieure 154.

Les moyens de déverrouillage permettent avantageusement de contrôler l'utilisation du distributeur par des enfants.

### Assemblage

L'assemblage d'un distributeur selon l'invention est particulièrement simple.

L'opérateur monte la tête de piston sur la tige de piston, en prenant en sandwich l'extrémité proximale 128 de l'étiquette indicatrice 126. Les pattes 106 sont encore fixées sur la tige de piston.

Il introduit le tenon du bouton-poussoir 78 dans le logement correspondant du curseur 44.

L'opérateur écarte ensuite les parties de pince 62a et 62b l'une de l'autre, puis introduit l'extrémité proximale de la tige de piston 38 entre ces parties de pince, jusqu'à ce que les parties de pince puissent prendre élastiquement appui entre les premières dents des deux rangées de dents et le plateau 104. Il relâche ensuite les parties de pince à cet effet

Après avoir introduit la tête de piston dans l'ouverture de la cuve 24, il insère l'ensemble formé par le bouton-poussoir, le curseur, le piston et la cuve entre les deux demi-coques 12a et 12b.

Les extrémités radialement extérieures 108a et 108b des pattes 106a et 106b, respectivement, sont insérées, de préférence encliquetées, dans les logements correspondant ménagés dans les demi-coques 12a et 12b, respectivement.

La présence des pattes d'inviolabilité 106a et 106b accélère considérablement le montage, en facilitant et en garantissant le positionnement correct des pions de butée, mais aussi de la tige de piston entre les nervures.

L'opérateur fixe ensuite les demi-coques 12a et 12b l'une à l'autre.

Il fixe ensuite l'embout d'application 16 sur la coque 12 en introduisant la canule dans la lumière d'embout 144 et en prenant en sandwich le clapet anti-retour 148 entre l'extrémité distale 150 de la canule et l'extrémité distale 146 de la tétine 142.

Les nervures de détrompage 170 à la surface extérieure de la canule de la cuve, les rainures longitudinales 169 à l'intérieur de la tétine et les échancrures 172 de la coque permettent de s'assurer du positionnement angulaire correct de l'embout d'application 16 et de la cuve par rapport à la coque 12.

Par ailleurs, pour fabriquer le capot, la jupe extérieure 154 est soudée sur la voûte transversale 156, la position angulaire, autour de l'axe X étant prédéterminée par la coopération du détrompeur 164 de la voûte transversale 156 et du bord rectiligne 166 du rebord distal 168 de la jupe extérieure 154. Il suffit alors de visser le capot pour fermer l'orifice d'éjection et activer le verrouillage de sécurité.

Le remplissage de la cuve avec le produit à doser peut être effectué par tous les moyens connus, par exemple avant d'introduire la tête de piston dans la cuve.

Dans un mode de réalisation préféré, les pattes 106a et 106b ne sont utilisées que pour faciliter le montage, comme décrit ci-dessus. Elles sont donc de préférence désactivées avant commercialisation du distributeur.

De préférence, le distributeur est fabriqué suivant les étapes a) à c) décrites ci-dessus.

A l'étape a), les différentes pièces constitutives du distributeur sont assemblées, de préférence comme décrit précédemment. Le réservoir est rempli, au moins partiellement, avec un produit liquide, de préférence un produit vétérinaire. Les techniques à cet effet sont connues. En particulier, le réservoir peut être rempli à travers son ouverture proximale 86, avant mise en place du piston, ou à travers la canule 26, avant ou après mise en place du piston.

A l'étape b), une pression, de préférence une unique pression, est exercée sur le bouton-poussoir 48, selon l'axe X. Cette pression provoque la rupture des zones de jonction des pattes 106a et 106b avec le plateau 104, puis le déplacement du poussoir 22 et du piston 20, dans le sens d'avancement. L'avancement du piston 20 pousse du produit dans la canule 26.

De préférence, la lumière de canule 28 n'est pas entièrement remplie de produit lors de l'étape a) et le remplissage de la cuve à l'étape a) est effectué de manière que l'avancement du piston à l'étape b) remplisse, au moins partiellement, de préférence complètement le volume vide de cette lumière.

Le complet remplissage de la lumière de canule 28 permet au distributeur d'être opérationnel, c'est-à-dire de délivrer une dose de produit, dès la pression suivante sur le bouton-poussoir. L'amorçage permet également de s'assurer que la première dose délivrée aura le même volume que les suivantes, ce qui est particulièrement avantageux pour des produits ayant un effet thérapeutique ou prophylactique.

De préférence encore, le volume de vide résiduel dans la lumière de canule 28, en fin d'étape a), est égal au volume de produit poussé dans ladite canule lors de l'étape b). Avantageusement, aucun produit n'est donc gaspillé lors de l'étape b).

A l'étape c), le distributeur est conditionné dans un emballage adapté à sa commercialisation. Il peut être disposé dans un emballage individuel, par exemple une boîte, de préférence couverte d'un film d'emballage, ou un blister. Il peut être disposé dans un emballage collectif, par exemple placé dans un carton avec d'autres distributeurs.

L'emballage est de préférence hermétique afin que l'utilisateur puisse s'assurer que le distributeur acheté est neuf.

### Utilisation

L'utilisation du distributeur se déduit directement de la description qui précède. Le distributeur est initialement dans la position représentée sur la figure 2.

L'opérateur appuie sur les régions de verrouillage 162 de manière à comprimer la jupe extérieure 154 et à déverrouiller les moyens de verrouillage. Il peut alors dévisser le capot. Le dévissage du capot dégage le téton 158 de l'orifice d'éjection.

L'opérateur applique alors la protubérance 160 de la tétine 142 sur la peau de l'animal. La forme arrondie de cette protubérance limite les risques de blessure. Comme pour l'actionnement d'une seringue, il appuie alors sur le bouton-poussoir 48, en retenant le distributeur au moyen de son index et de son majeur, disposés derrière les oreilles 13a et 13b, du côté distal, à la manière d'une seringue.

Le déplacement du bouton-poussoir 48 selon l'axe X est pratique puisqu'il permet la distribution de produit avec une seule main. Cette caractéristique est particulièrement avantageuse lorsque le produit doit être appliqué sur la peau d'un animal nerveux.

Si la désactivation n'a pas déjà été effectuée en usine, les pattes 106 sont encore fixées au plateau 104 au moment de la première utilisation du distributeur. L'avancement du bouton-poussoir est donc entravé par les zones de jonction entre le plateau 104 et les pattes 106. L'opérateur doit donc exercer une pression relativement élevée sur le bouton-poussoir 48 afin de faire céder ces zones de jonction. Le bouton-poussoir constitue ainsi un organe de désactivation des pattes.

Après la première pression sur le bouton-poussoir 48, les pattes sont ainsi désolidarisées du plateau 104 et désactivées de manière irréversible. Elles n'entravent plus le déplacement de la tête de piston dans la cuve sous l'effet des pressions successives sur le bouton-poussoir 48.

La rupture des zones de jonction des pattes peut avantageusement s'effectuer dans le même mouvement que la première distribution d'une dose. L'utilisation du distributeur est donc plus efficace et plus pratique.

Après désactivation des pattes, le bouton-poussoir 48 peut avancer dans le sens d'avancement A, entraînant avec lui la base 50 du curseur 44. L'appui des branches de ressort 52 sur les épaulements 56 s'opposent à cet avancement. Cependant, les branches de ressort 52 présentent une souplesse qui leur permet de se courber élastiquement vers l'axe X, et ainsi autoriser l'avancement de la base 50.

Les parties de pince sont par ailleurs en contact avec le plateau 104. L'avancement de la base 50 conduit donc à l'avancement de la tête de piston dans la cuve. La surpression résultant de cet avancement conduit à une ouverture du clapet anti-retour 148. Le produit peut donc être éjecté hors du distributeur, par l'orifice d'éjection.

L'avancement de la tête de piston se poursuit jusqu'à ce que les bras de butée 70a et 70b entrent en butée avec les pions de butée 110a et 110b, respectivement.

L'opérateur relâche alors la pression sur le bouton-poussoir 48. Sous l'effet de l'élasticité des branches de ressort 52a et 52b, le curseur 44, et donc le bouton-poussoir 48 reculent, suivant le sens de recul R, jusqu'à leur position initiale, c'est-à-dire jusqu'à ce que le bord périphérique 80 du bouton-poussoir 48 entre en butée avec le rebord annulaire 82 de la coque.

Lors de ce mouvement de recul, les parties de pince 62a et 62b glissent sur les faces inclinées 68a et 68b des dents les plus distales des deux rangées de dents, dites "premières dents". L'élasticité des branches de cliquet 58a et 58b permet avantageusement aux parties de pince 62a et 62b de s'écarter de l'axe X, en glissant sur les faces inclinées 68a et 68b des premières dents. Ce glissement ne permet pas de s'opposer à l'action de recul produite par les branches de ressort 52a et 52b. Ce glissement ne permet pas non plus de faire reculer le piston, maintenu en position par les frottements du joint 116 sur la surface intérieure de la cuve, mais aussi, avantageusement, par la fermeture du clapet anti-retour 148 qui entrave l'entrée d'air dans la cuve, et donc le recul du piston, le produit à l'intérieur de la cuve étant sensiblement incompressible.

Sensiblement à l'instant où le rebord périphérique 80 du bouton-poussoir entre en butée sur le rebord annulaire 82 de la coque 12, ou légèrement avant, les parties de pince 62a et 62b dépassent la limite proximale des faces inclinées 68a et 68b des premières dents sur lesquelles elles glissent, et, sous l'effet de l'élasticité des branches de cliquet, reviennent vers l'axe X, en se positionnant devant les faces de blocage 66a et 66b des premières dents.

Lors des mouvements de va-et-vient du bouton-poussoir, les nervures de guidage 76 assurent avantageusement un guidage efficace du curseur 44, mais aussi de la tige de piston 38.

Le distributeur est alors dans une position adaptée pour la distribution d'une deuxième dose.

Si l'opérateur ne doit pas délivrer une deuxième dose immédiatement, il revisse le capot 18 sur le filet de tétine. A la fin du vissage, les moyens de verrouillage sont activés automatiquement, ce qui évite toute ouverture par les enfants.

Par ailleurs, le téton 158 disposé au fond du capot pénètre dans la lumière d'embout 144, en venant comprimer, de manière étanche, le clapet anti-retour et/ou obturer, de manière étanche, l'orifice d'éjection. Aucun échappement de produit dans le capot, par exemple sous l'effet d'une agitation du distributeur, n'est donc possible, ce qui garantit la propreté.

En outre, la pénétration du téton évite que, lors de l'utilisation suivante, du produit affleure à l'orifice d'éjection, ce qui évite tout contact involontaire de produit avec la peau de l'opérateur.

La distribution de la deuxième dose s'effectue comme la distribution de la première dose. Le mouvement d'avancement s'effectue cependant par poussée des branches de cliquet sur la face de blocage des premières dents, et non plus sur le plateau 104.

A chaque distribution d'une dose, le piston avance sur une distance égale à la longueur d'une dent, dans l'exemple représenté.

La distribution des doses suivantes s'effectue comme la distribution de la deuxième dose, par poussée des branches de cliquet sur les faces de blocage des dents successives.

Les mouvements de va-et-vient du bouton-poussoir le long de l'axe X permettent ainsi la délivrance d'une pluralité de doses, toutes identiques, de manière simple et rapide.

Lors de la dernière course de dosage, la tête de piston vient se positionner contre le fond 122 de la cuve, assurant ainsi un vidage complet de la cuve. Les parties de pince 62 sont alors en contact avec les faces de blocage des dents les plus proximales, ou "dernières dents" des deux rangées de dents.

Le dispositif peut alors être mis au rebut.

Initialement, l'opérateur peut apercevoir, par la fenêtre 138, un chiffre, par exemple « 18 », lui indiquant le nombre de doses restant à distribuer. L'avancement de la tête de piston dans la cuve entraîne l'étiquette indicatrice 126 vers l'intérieur de la cuve et modifie la région de l'étiquette indicatrice visible à travers la fenêtre 138. Le chiffre visible décroit de préférence d'une unité à chaque distribution d'une dose. L'opérateur peut ainsi facilement évaluer le niveau de remplissage de la cuve, mais aussi vérifier que le traitement est correctement suivi.

Dans un mode de réalisation, lors de la première course de dosage, pendant laquelle les pattes sont séparées du plateau 104, le liquide expulsé de la cuve vient remplir la lumière de canule 28, initialement au moins en partie vide. Aucun produit n'est donc éjecté du distributeur.

Avantageusement, l'opérateur peut ainsi dissocier l'opération par laquelle il détache les pattes et les opérations suivantes par lesquelles il distribue du produit.

Dans ce mode de réalisation, les deux premières indications 137 sur l'étiquette indicatrice peuvent être identiques, par exemple égales à « 18 ». De préférence, la première indication 137 est cependant utilisée pour indiquer que les pattes n'ont pas encore été désactivées. Par exemple, une lettre « A » peut apparaitre dans la fenêtre avant leur désactivation.

Comme cela apparaît clairement à présent, l'invention fournit un distributeur compact, facile à utiliser, constitué d'un nombre très réduit de pièces, facile à assembler, ergonomique et pratique à utiliser, permettant d'évaluer le nombre de doses déjà délivrées, ou restant à délivrer, fiable, assurant des conditions d'hygiène et de sécurité optimales et d'un coût réduit.

Bien entendu, l'invention n'est pas limitée aux modes de réalisation décrits et représentés, fournis à des fins illustratives seulement.

En particulier, les pattes pourraient être fixées sur une autre partie du piston que le plateau 104, par exemple sur le support de joint 114.

Le mouvement de recul du curseur peut conduire à un glissement des parties d'appui successivement sur plusieurs dents successives. De préférence cependant, la course de dosage correspond à un glissement sur une seule dent.

Les dents pourraient être remplacées par des trous, traversant ou non, pourvu que la forme des parties d'appui soit adaptée en conséquence, par exemple pour prendre la forme de crochets ou de griffes accrochant le bord desdits trous dans le sens d'avancement et sortant desdits trous dans le sens de recul.

La cuve pourrait être amovible.

La coque est optionnelle dans la mesure où elle pourrait, par exemple, être remplacée par un réservoir présentant une forme adaptée.

Le ressort pourrait prendre une autre forme, et en particulier être hélicoïdal, à air comprimé, à spires jointives, en accordéon, élastique...

## Revendications

1. Distributeur multi-doses comportant :
- un réservoir de produit (14), d'axe X, débouchant à une extrémité distale (29) par un orifice de sortie (32), et comportant une cuve (24) ;
- un piston (20) comportant
- une tige de piston (38) portant une crémaillère (74a, 74b) et,
- à une extrémité distale de la tige de piston, une tête de piston (34) montée coulissante dans la cuve (24), selon l'axe X ;
- un cliquet (62a,62b) monté mobile, selon l'axe X, par rapport au réservoir, et configuré pour coopérer avec ladite crémaillère de manière à n'entraîner le piston, selon l'axe X, que dans un sens d'avancement du piston dans la cuve ;
- un ressort (51) disposé de manière à repousser le cliquet, selon l'axe X, dans un sens de recul opposé au sens d'avancement, le ressort et le cliquet formant un ensemble monobloc,
- une coque contenant le cliquet et au moins une partie du piston ;
- un bouton-poussoir (48) faisant saillie hors de la coque et monté mobile, selon l'axe X, par rapport à la coque, le bouton-poussoir étant couplé mécaniquement avec le cliquet de manière qu'une pression sur ledit bouton-poussoir dans le sens d'avancement fasse avancer le cliquet dans le sens d'avancement.

2. Distributeur selon la revendication 1, comportant un clapet anti-retour (148), disposé de manière à obturer l'orifice de sortie et à n'autoriser qu'une sortie de produit hors du réservoir.

3. Distributeur selon l'une quelconque des revendications précédentes, dans lequel le ressort s'étend dans un plan incluant l'axe X et comporte au moins une branche de ressort divergeant de l'axe X, suivant le sens d'avancement.

4. Distributeur selon la revendication immédiatement précédente, dans lequel la branche de ressort est disposée entre deux nervures de guidage (76ₐ₁,76ₐ₂,76_{b1},76_{b2}) configurées pour guider la déformation de la branche de ressort dans un plan passant par l'axe X.

5. Distributeur selon l'une quelconque des revendications précédentes, dans lequel la crémaillère comporte des première et deuxième rangées de dents (64a,64b), lesdites rangées de dents s'étendant selon l'axe X, et le cliquet comportant des première et deuxième branches de cliquet (58a,58b) terminées par des première et deuxième parties de pince (62a,62b), respectivement, disposées de manière à pincer élastiquement ladite crémaillère en prenant appui sur lesdites première et deuxième rangées de dents, respectivement.

6. Distributeur selon la revendication immédiatement précédente, dans lequel chaque branche de cliquet est disposée entre deux nervures de guidage (76ₐ₁,76ₐ₂; 76_{b1},76_{b2}) configurées pour guider la déplacement de ladite branche de cliquet dans un plan passant par l'axe X, l'écartement des deux nervures de guidage de chaque couple de nervures de guidage étant sensiblement égal à la largeur d'une branche de cliquet respective, de manière à guider la déformation et/ou le déplacement de ladite branche de cliquet.

7. Distributeur selon l'une quelconque des revendications précédentes, comportant un organe de blocage (106a,106b) désactivable, configuré pour empêcher la distribution d'un produit contenu dans le réservoir, et disposé à l'intérieur de la coque.

8. Distributeur selon la revendication immédiatement précédente, dans lequel l'organe de blocage (106a,106b) est désactivable de manière irréversible, de préférence sécable.

9. Distributeur selon l'une quelconque des revendications précédentes, dans lequel la tige de piston (38) et l'organe de blocage (106) constituent une pièce monobloc.

10. Distributeur selon l'une quelconque des trois revendications immédiatement précédentes, dans lequel l'organe de blocage comporte un pion de butée (110a,110b) configuré pour limiter le déplacement du cliquet selon l'axe X, dans le sens d'avancement, après désactivation.

11. Distributeur selon l'une quelconque des revendications précédentes, comportant une étiquette indicatrice (126) fixée, par une extrémité proximale (128), sur le piston (20) et présentant une extrémité distale (130) libre disposée contre la surface extérieure (132) de la cuve (24).

12. Distributeur selon la revendication immédiatement précédente, dans lequel la coque ménage une fenêtre (138) à travers laquelle une portion de l'étiquette indicatrice est visible depuis l'extérieur de la coque.

13. Distributeur selon l'une quelconque des revendications précédentes, dans lequel l'étiquette indicatrice porte des indications (137), le pas entre deux indications successives, le long de l'axe X, étant constant et égal à la distance maximale que le cliquet peut parcourir le long de l'axe X dans le sens d'avancement.

14. Distributeur selon l'une quelconque des revendications précédentes, dans lequel la cuve contient un produit vétérinaire, liquide ou pâteux.

15. Procédé de fabrication d'un distributeur conditionné dans un emballage, ledit procédé comportant les étapes successives suivantes :
a) assemblage des différentes pièces constitutives d'un distributeur selon l'une quelconque des revendications 7 à 14, et remplissage de la cuve avec un produit liquide ;
b) désactivation de l'organe de blocage ;
c) conditionnement dudit distributeur dans un emballage.

## Patentansprüche

1. Mehrdosenspender, umfassend:
- einen Produktbehälter (14) mit X-Achse, der durch einen Auslass (32) in ein distales Ende (29) mündet und ein Gefäß (24) beinhaltet;
- einen Kolben (20), umfassend
- einen Kolbenschaft (38) mit einer Zahnstange (74a, 74b) und
- einen an einem distalen Ende des Kolbenschafts entsprechend der X-Achse gleitend montierten Kolbenkopf (34) in dem Gefäß (24);
- eine entsprechend der X-Achse im Verhältnis zum Behälter beweglich montierte Sperrklinke (62a, 62b) und darauf ausgelegt, mit der Zahnstange zusammenzuwirken, damit der Kolben entsprechend der X-Achse nur in einer Vorwärtsrichtung des Kolbens im Gefäß angetrieben wird;
- eine Feder (51), die so angeordnet ist, dass die Sperrklinke entsprechend der X-Achse in einer der Vorwärtsrichtung entgegengesetzten Rückstoßrichtung zurückgeschoben wird, wobei Feder und Sperrklinke einen gemeinsamen Monoblock bilden,
- ein Gehäuse, welches den Verschluss und mindestens einen Teil des Kolbens beinhaltet;
- einen außerhalb des Gehäuses vorstehenden und entsprechend der X-Achse im Verhältnis zum Gehäuse beweglich montierten Drucktaster (48) wobei der Drucktaster mechanisch mit der Sperrklinke gekoppelt ist, sodass ein Druck auf den Drucktaster in Vorwärtsrichtung bewirkt, dass sich die Sperrklinke in Vorwärtsrichtung verschiebt.

2. Spender nach Anspruch 1, mit einem Rückschlagventil (148), dass so angeordnet ist, dass der Auslass abgedeckt und nur eine Produktabgabe außerhalb des Behälters möglich ist.

3. Spender nach einem der vorhergehenden Ansprüche, wobei sich die Feder in einer Ebene mit der X-Achse erstreckt und mindestens eine von der X-Achse abweichende Federwelle in Vorwärtsrichtung umfasst.

4. Spender nach dem unmittelbar vorhergehenden Anspruch, wobei die Federwelle zwischen zwei Führungsrippen angeordnet ist (76ₐ₁, 76ₐ₂, 76_{b1}, 76_{b2}), die darauf ausgelegt sind, die Verformung der Federwelle in einer Ebene durch die X-Achse zu führen.

5. Spender nach einem der vorhergehenden Ansprüche, wobei die Zahnstange erste und zweite Zahnreihen (64a, 64b) umfasst, wobei sich die Zahnreihen entsprechend der X-Achse erstrecken, und die Sperrklinke, die erste und zweite Klinkenzahnreihen (58a, 58b) umfasst, die in ersten bzw. zweiten Klemmteilen (62a, 62b) enden, die entsprechend angeordnet sind, um die Zahnstange durch Aufliegen auf der ersten bzw. zweiten Zahnreihe elastisch zu klemmen.

6. Spender nach dem unmittelbar vorhergehenden Anspruch, wobei jeder Sperrklinkenbügel zwischen zwei Führungsrippen angeordnet ist (76ₐ₁, 76ₐ₂; 76_{b1}, 76_{b2}), die darauf ausgelegt sind, um die Bewegung dieses Klinkenbügels in einer Ebene durch die X-Achse zu führen, wobei der Abstand der beiden Führungsrippen jedes einzelnen Führungspaares deutlich auf die Breite eines jeweiligen Sperrklinkenbügels ausgerichtet ist, sodass die Verformung und/oder das Verschieben dieses Sperrklinkenbügels geführt wird.

7. Spender nach einem der vorhergehenden Ansprüche mit einer deaktivierbaren Sperrvorrichtung (106a, 106b), die darauf ausgelegt ist, das Spenden eines im Behälter enthaltenen Produkts zu verhindern, und die im Inneren des Gehäuses angeordnet ist.

8. Spender nach dem unmittelbar vorhergehenden Anspruch, wobei die Sperrvorrichtung (106a, 106b) irreversibel, vorzugsweise teilbar, deaktiviert werden kann.

9. Spender nach einem der vorhergehenden Ansprüche, wobei der Kolbenschaft (38) und die Sperrvorrichtung (106) einen Monoblock darstellen.

10. Spender nach einem der drei unmittelbar vorhergehenden Ansprüche, wobei die Sperrvorrichtung einen Anschlagkolben (110a, 110b) umfasst, der darauf ausgelegt ist, die Verschiebung der Sperrklinke entsprechend der X-Achse nach Deaktivierung in Vorwärtsrichtung zu begrenzen.

11. Spender nach einem der vorhergehenden Ansprüche mit einem Hinweisetikett (126), das mit einem proximalen Ende (128) am Kolben (20) befestigt ist und ein freies distales Ende (130) hat, das gegen die Außenfläche (132) des Gefäßes (24) angeordnet ist.

12. Spender nach dem unmittelbar vorhergehenden Anspruch, wobei das Gehäuse ein Fenster (138) hat, durch das ein Teil des Hinweisetiketts von der Außenseite des Gehäuses sichtbar ist.

13. Spender nach einem der vorhergehenden Ansprüche, wobei das Hinweisetikett Angaben (137) enthält, wobei der Abstand zwischen zwei aufeinanderfolgenden Angaben entlang der X-Achse konstant und gleich dem Höchstabstand ist, den die Sperrklinke entlang der X-Achse in Vorwärtsrichtung zurücklegen kann.

14. Spender nach einem der vorhergehenden Ansprüche, wobei das Gefäß ein veterinärmedizinisches, flüssiges oder pastöses Produkt enthält.

15. Verfahren zur Herstellung eines in einer Verpackung verpackten Spenders, wobei das Verfahren die folgenden aufeinanderfolgenden Schritte umfasst:
a) Montieren der einzelnen Bestandteile eines Spenders nach einem der Ansprüche 7 bis 14 und Befüllen des Gefäßes mit einem flüssigen Produkt;
b) Deaktivieren der Sperrvorrichtung;
c) Verpacken des Spenders in einer Verpackung.

## Claims

1. A multi-dose dispenser comprising:
- a product reservoir (14), having an X-axis, draining from a distal end (29) via a discharge port (32), and comprising a tank (24);
- a piston (20) comprising
- a piston rod (38) bearing a rack (74a, 74b) and,
- at a distal end of the piston rod, a piston head (34) mounted sliding into the tank (24) along the X axis;
- a mobile ratchet (62a, 62b) mounted along the X-axis relative to the reservoir and configured to cooperate with said rack in such a way as to move the piston only forward along the X-axis into the tank;
- a spring (51) arranged so as to push the ratchet backward along the X axis in the opposite direction to that of the forward movement, the spring and the ratchet forming a monobloc assembly,
- a shell containing the ratchet and at least a portion of the piston;
- a push button (48) protruding from the shell and mounted so as to move along the X-axis relative to the shell, the push button being mechanically coupled to the ratchet such that pressing on said push button in the direction of the forward movement causes the ratchet to move forward.

2. A dispenser according to claim 1, comprising a check valve (148) arranged to seal off the discharge port and to allow product discharge only from the tank.

3. A dispenser according to any of the preceding claims, wherein the spring extends into a plane including the X-axis and comprises at least one spring limb diverging from the X-axis in the forward direction.

4. A dispenser according to the preceding claim, wherein the spring limb is arranged between two guide ribs (76ₐ₁, 76ₐ₂, 76_{b1}, 76_{b2}) configured to guide the deformation of the spring limb into a plane passing through the X-axis.

5. A dispenser according to any of the preceding claims, wherein the rack comprises first and second rows of teeth (64a, 64b), said rows of teeth extending along the X-axis, and the ratchet comprising first and second ratchet legs (58a, 58b) ending in first and second gripper parts (62a, 62b), respectively, arranged so as to elastically grip said rack by resting on said first and second rows of teeth, respectively.

6. A dispenser according to the preceding claim, wherein each ratchet leg is arranged between two guide ribs (76ₐ₁,76ₐ₂; 76_{b1},76_{b2}) configured to guide the movement of said ratchet leg in a plane passing through the X-axis, the gap between the two guide ribs of each pair of guide ribs being substantially equal to the width of a respective ratchet leg, so as to guide the deformation and/or movement of said ratchet leg.

7. A dispenser according to any of the preceding claims, comprising a deactivatable locking element (106a, 106b) configured to prevent the dispensing of a product contained in the reservoir, and arranged inside the shell.

8. A dispenser according to the preceding claim, wherein the locking element (106a, 106b) can be irreversibly deactivated, preferably scored.

9. A dispenser according to any of the preceding claims, wherein the piston rod (38) and the locking element (106) constitute a monobloc part.

10. A dispenser according to any of the three preceding claims, wherein the locking element comprises a stop peg (110a, 110b) configured to limit the forward movement of the ratchet along the X-axis, after deactivation.

11. A dispenser according to any of the preceding claims,
comprising an indicator label (126) attached by a proximal end (128) to the piston (20) and having a free distal end (130) against the outer surface (132) of the tank (24).

12. A dispenser according to the preceding claim, wherein the shell has a window (138) through which a portion of the indicator label is visible from outside of the shell.

13. A dispenser according to any of the preceding claims, wherein the indicator label bears indications (137), the step between two successive indications along the X axis being constant and equal to the maximum distance that the ratchet can move forward along the X axis.

14. A dispenser according to any of the preceding claims, wherein the tank contains a veterinary product, in liquid or paste form.

15. A method of manufacturing a dispenser packaged in a package, said method comprising the following successive steps:
a) assembly of the various constituent parts of a dispenser according to any of claims 7 to 14, and filling the tank with a liquid product;
b) deactivating the locking element;
c) packaging of said dispenser in a package.
